# EUROPEAN PATENT APPLICATION

(11) **EP 4 151 722 A1**
(43) Date of publication of application: **22.03.2023**
(21) Application number: 22191002.9
(22) Date of filing: 15.11.2019
(51) Int. Cl.: C12N 5/0783, C07K 14/705, C12N 15/87, A61K 48/00, C07K 14/725

(54) **IMPROVED T CELL MANUFACTURING PROCESS**

(30) Priority: 16.11.2018 US 201862768579 P
(62) Divisional of application: 19821337.3
(71) Applicant: Celgene Corporation, Summit, NJ 07901 (US)
(72) Inventor: BRIEVA, Thomas, A., Manalapan, 07726 (US); HSIUNG, David, Jersey City, 07302 (US); JONES, Seth, Bound Brook, 08805 (US); MISTRY, Shiv, Clifton, 07013 (US); RAJAEI, Nayyereh, Franklin Park, 08823 (US)
(74) Representative: Carpmaels & Ransford LLP

(57) **Abstract**

Provided herein are improved methods of manufacturing cells, including T cells and CAR T cells. Also provided herein are methods of manufacturing cells, such as T cells and CAR T cells, obtainable from blood using a method incorporating membrane filtration and Ammonium-Chloride-Potassium (ACK) buffer to isolate the cells from other blood components.

## Description

### CROSS-REFERENCE TO RELATED APPLICATIONS

This application claims the benefit of U.S. Provisional Patent Application No. 62/768,579 filed November 16, 2018, the entire content of which is incorporated herein by reference in its entirety.

### 1. FIELD

The present invention relates to the field of medicine, in particular the production of cellular therapies for the treatment of cancer.

### 2. BACKGROUND

Immunotherapy based on T cells expressing an artificial receptor, *e.g.,* a chimeric antigen receptor or T cell receptor (TCR), polypeptides that target the T cells to a particular tumor-associated antigen, and provides primary and generally costimulatory signaling to activate and increase proliferation of the T cell, has become an increasingly promising treatment modality, particularly for patients who have exhausted other lines of treatment. However, for autologous CAR T cell therapy, the manufacture of the cells is still laborious and time-intensive. As a result, there is a need in the field for improved methods of manufacturing CAR T cells that will decrease the time and expense of producing such cells. Provided herein is such an improved method.

### 3. SUMMARY

In a first aspect, provided herein are methods of manufacturing cells obtainable from blood using a method incorporating membrane filtration and Ammonium-Chloride-Potassium (ACK) buffer to isolate the cells from other blood components. In a first embodiment, provided herein is a method of manufacturing cells from peripheral blood mononuclear cells (PBMCs) from a subject from which a blood sample is obtained, comprising the steps of: (a) obtaining blood cells from the blood sample, e.g., using leukapheresis or blood draw, to obtain peripheral blood mononuclear cells (PBMCs); (b) optionally freezing the blood cells and obtaining the PBMCs after thaw, or optionally obtaining and freezing the PBMCs, followed by thawing prior to subsequent steps; (c) isolating the PBMCs using a membrane filtration system and Ammonium-Chloride-Potassium (ACK) buffer; (d) washing the PBMCs via centrifugation; (e) optionally cryopreserving the PBMCs; (f) optionally thawing the PBMCs if the PBMCs are cryopreserved in step (e); (g) washing the PBMCs using a membrane filtration system; (h) manufacturing the cells from the PBMCs; and (i) washing the cells using a membrane filtration system. In a specific embodiment, steps (a)-(h) in said method are performed in order. In a specific embodiment, the cells are chimeric antigen receptor (CAR)-expressing T cells (CAR T cells).

In another embodiment, provided herein is a method of manufacturing chimeric antigen receptor (CAR)-expressing T cells (CAR T cells) from peripheral blood mononuclear cells (PBMCs) from a subject from which a blood sample is obtained, comprising the steps of: (a) obtaining PBMCs from the blood sample; (b) isolating PBMCs that have been obtained from the blood sample using a membrane filtration system and Ammonium-Chloride-Potassium (ACK) buffer; (c) washing the PBMCs via centrifugation; (d) optionally cryopreserving the PBMCs; (e) optionally thawing the PBMCs cryopreserved in step (d); (f) washing the PBMCs using a membrane filtration system; (g) manufacturing the CAR T cells from the PBMCs from step (f); and (h) washing the CAR T cells from step (g) using a membrane filtration system. In a specific embodiment, steps (a)-(h) in said method are performed in order.

In another embodiment, provided herein is a method of manufacturing chimeric antigen receptor (CAR)-expressing T cells (CAR T cells) from peripheral blood mononuclear cells (PBMCs) from a subject from which a blood sample is obtained, comprising the steps of: (a) isolating PBMCs that have been obtained from a blood sample form the subject, wherein the said isolating uses a membrane filtration system and Ammonium-Chloride-Potassium (ACK) buffer; (b) washing the PBMCs via centrifugation; (c) optionally cryopreserving the PBMCs; (d) optionally thawing the PBMCs cryopreserved in step (c); (e) washing the PBMCs using a membrane filtration system; (f) manufacturing the CAR T cells from the PBMCs from step (f); and (g) washing the CAR T cells from step (f) using a membrane filtration system. In a specific embodiment, steps (a)-(g) in said method are performed in order.

In another embodiment, provided herein is a method of manufacturing cells from peripheral blood mononuclear cells (PBMCs) from a blood sample from a subject, comprising the steps of: (a) isolating PBMCs from a blood sample from the subject, wherein the blood sample is a leukapheresed blood sample or is from a blood draw, using a membrane filtration system and Ammonium-Chloride-Potassium (ACK) buffer; (b) optionally cryopreserving the PBMCs from step (a); (c) optionally thawing the PBMCs from step (b); (d) washing the thawed PBMCs from step (c) using a membrane filtration system; (e) manufacturing the cells from the PBMCs from step (d) ; and (f) washing the cells from step (e) using a membrane filtration system. In a specific embodiment, steps (a)-(f) in said method are performed in order. In a specific embodiment, the cells are chimeric antigen receptor (CAR)-expressing T cells (CAR T cells).

In another embodiment, provided herein is a method of manufacturing cells from peripheral blood mononuclear cells (PBMCs) from a blood sample from a subject, comprising the steps of: (a) isolating PBMCs from a blood sample from the subject, wherein the blood sample is a leukapheresed blood sample or is from a blood draw, using a membrane filtration system and Ammonium-Chloride-Potassium (ACK) buffer; (b) cryopreserving the PBMCs from step (a); (c) thawing the PBMCs from step (b); (d) washing the thawed PBMCs from step (c) using a membrane filtration system; (e) manufacturing the cells from the PBMCs from step (d) ; and (f) washing the cells from step (e) using a membrane filtration system. In a specific embodiment, steps (a)-(f) in said method are performed in order. In a specific embodiment, the cells are chimeric antigen receptor (CAR)-expressing T cells (CAR T cells).

In another embodiment, provided herein is a method of manufacturing cells from peripheral blood mononuclear cells (PBMCs) from a blood sample from a subject, comprising the steps of: (a) isolating PBMCs from a blood sample from the subject using a membrane filtration system and Ammonium-Chloride-Potassium (ACK) buffer; (b) optionally cryopreserving and thawing the PBMCs from step (a); (c) optionally washing the thawed PBMCs from step (b) using a membrane filtration system; (d) manufacturing the cells from the PBMCs; and (e) washing the cells using a membrane filtration system. In a specific embodiment, steps (a)-(e) in said method are performed in order. In a specific embodiment, the blood sample is a leukapheresed blood sample. In a specific embodiment, the cells are chimeric antigen receptor (CAR)-expressing T cells (CAR T cells).

In another embodiment, provided herein is a method of manufacturing cells from peripheral blood mononuclear cells (PBMCs) from a blood sample from a subject, comprising the steps of: (a) isolating PBMCs from a blood sample from the subject using a membrane filtration system and Ammonium-Chloride-Potassium (ACK) buffer; (b) cryopreserving and thawing the PBMCs from step (a); (c) washing the thawed PBMCs from step (b) using a membrane filtration system; (d) manufacturing the cells from the PBMCs; and (e) washing the cells using a membrane filtration system. In a specific embodiment, steps (a)-(e) in said method are performed in order. In a specific embodiment, the blood sample is a leukapheresed blood sample. In a specific embodiment, the cells are chimeric antigen receptor (CAR)-expressing T cells (CAR T cells).

In specific embodiments of any of the above embodiments, the cells are T cells (T lymphocytes), natural killer cells (NK cells) or dendritic cells. In specific embodiments, the T cells are cytotoxic T lymphocytes (CTLs), CD4+ T cells, CD8+ T cells, or T central memory (T_{cm}) cells. In specific embodiments, the cells, *e.g.,* T cells, have been genetically modified to express a polypeptide, *e.g.,* a chimeric receptor. In more specific embodiments, the chimeric receptor is a T cell receptor (TCR) or chimeric antigen receptor. In more specific embodiments, the cells of any of the above methods are chimeric antigen receptor-expressing T cells (CAR T cells).

In specific embodiments of any of the above embodiments, at least one, or all, of the membrane filtration system(s) is a tangential flow filtration system. In specific embodiments of any of the above embodiments, at least one, or all, of the membrane filtration system(s) may be a spinning membrane filtration system. In a specific embodiment of any of the above embodiments, the membrane filtration system is spinning membrane filtration system such as a LOVO Automated Cell Processing System. In another specific embodiment of any of the above embodiments, wherein the ACK buffer comprises 50-300 mM ammonium chloride, 5-25 mM potassium carbonate, and 0.05-0.25 mM sodium EDTA. In a more specific embodiment, said ACK buffer comprises 150 mM ammonium chloride, 10 mM potassium carbonate, and 0.1 mM sodium EDTA. In another specific embodiment of any of the above embodiments said method improves reduction of platelets and red blood cells from said PBMCs by 18%-36% as compared to the same method using density gradient centrifugation in place of each use of spinning membrane filtration. In another specific embodiment of any of the above embodiments said method improves reduction of platelets and red blood cells from said PBMCs by 10%-50%, 15%-45%, 20%-40%, 20%-35%, 20%-30%, 25%-35%, 25%-30%, 25%-40%, or 30-35% as compared to the same method using density gradient centrifugation in place of each use of spinning membrane filtration. In another specific embodiment of any of the above embodiments, said method improves recovery of the cells, *e.g.,* CAR T cells, after T cell manufacturing by 17%-36% as compared to the same method using density gradient centrifugation in place of each use of spinning membrane filtration. In another specific embodiment of any of the above embodiments, said method improves recovery of the cells, *e.g.,* CAR T cells, after T cell manufacturing by about 10%-50%, 15%-45%, 20%-40%, 20%-35%, 20%-30%, 25%-35%, 25%-30%, 25%-40%, or 30-35%, as compared to the same method using density gradient centrifugation in place of each use of spinning membrane filtration.

### 4. DETAILED DESCRIPTION

### 4.1. Improved Process for Producing Cells - Overview

Provided herein is a process for manufacturing cells that incorporates an improved process for producing the cells. The manufacturing process starts with isolation of autologous peripheral blood mononuclear cells (PBMCs) from a patient's leukapheresis or from a blood draw.

The manufacturing process begins with the isolation of PBMC from a patient's leukapheresis or from a blood draw. The autologous PBMCs are isolated from the leukapheresis or from a blood draw via a membrane filtration system, for example, a spinning membrane filtration system, e.g., with a pore size of 3.6 µm, *e.g.,* to remove platelets and cell debris. After platelet and cell debris removal, an ammonium-chloride-potassium (ACK) buffer is used to lyse red blood cells. The PBMCs are then separated from the ACK buffer solution and washed via centrifugation. This combination of spinning membrane filtration followed by ACK lysis of RBCs represents a first improvement over a prior process, which used density gradient centrifugation (Cell-Saver 5+ (CS5+; Haemonetics, Braintree, Massachusetts)).

After wash, the isolated PBMCs are resuspended and then formulated in a suitable solution, e.g., Cryostor. Alternatively, T cells (or NK cells or dendritic cells) may be isolated from the PBMCs prior to formulation in such a solution. For T cells, magnetic beads, such as Dynabeads, coated with anti-CD3, anti-CD4 or anti-CD8 antibodies may be used for T cell isolation. The formulated PBMCs or isolated cells may then be frozen using a controlled rate freezer, stored, and released. This step may be followed by a cell culture step. For example, a 37°C water bath may be used on what is defined as Day 0 to thaw the cryopreserved PBMCs or isolated cells prior to the cells being washed and resuspended in growth medium suitable for the growth of such cells, using a membrane filtration system, e.g., a spinning membrane filtration system, or centrifugation. A preferred T cell growth medium (TCGM) comprises 93% (v/v) cell culture medium X-VIVO-15 (Lonza), 10mM HEPES, 2 mM GlutaMAX^{™}, 5% (v/v) human AB serum, and 100 IU/mL recombinant human interleukin-2 (rhIL-2) (*see* Hollyman et al, J Immunother 2009, 32:169-180).

The culture may be initiated and activated by seeding isolated PBMCs into a gas permeable cell differentiation bag at a concentration of 1 × 10⁶ PBMC/mL in medium, e.g., TCGM, supplemented with activation reagents (*e*.*g*., for T cells, Dynabeads coated with anti-CD3 and anti-CD28). The cells may then then incubated at 37°C, preferably in air with 5% CO_{2.} In the case of CAR T cells, such incubation may continue until transduction of the T cells with a CAR-expressing vector. T cell activation can be measured by an increase in cell size (cell blasting) and cell clustering. Cell size (an indicator of activation) may be monitored following culture initiation using, *e.g.,* the Coulter method.

For the manufacture of CAR T cells, transduction with CAR-expressing vector may be performed on by diluting a specified volume of the vector in medium, e.g., TCGM, which is then added to the cell culture. The volume of vector may be selected based on the number of PBMC seeded on day 0, the viral titer of the vector lot used, and the target multiplicity of infection (MOI). The target MOI in certain embodiments is specific to the lot of vector and is selected to achieve comparable results batch-to-batch. The cells are then incubated at 37°C with 5% CO₂.

After transduction with the vector, the culture is incubated and allowed to expand for a period of time and at a target seeding density. The culture may then be reseeded into, *e.g.,* gas permeable cell expansion bags or other culture bag, such as a WAVE^{™} bioreactor, and incubated.

Thereafter, *e.g.,* on day 10, the cells are harvested and washed using a membrane filtration system, *e*.*g*., a spinning membrane filtration system, followed by aseptic transfer, using a closed system, of the culture into a processing bag for formulation into the final drug product. This represents a third improvement over the prior, baseline process, which used a CS5+ device to perform this last wash.

For any of the above steps using a spinning membrane filter, a preferred apparatus is the LOVO Automated Cell Processing System (Fresenius Kabi).

In a specific embodiment, provided herein is a method of manufacturing CAR T cells from peripheral blood mononuclear cells (PBMCs) from a subject from which a blood sample is obtained, comprising the steps of: (a) obtaining blood cells from the blood sample, e.g., using leukapheresis or blood draw to obtain peripheral blood mononuclear cells (PBMCs); (b) optionally freezing the blood cells and obtaining the PBMCs after thaw, or optionally obtaining and freezing the PBMCs, followed by thawing prior to subsequent steps; (c) isolating the PBMCs using a membrane filtration system, e.g., a spinning membrane filtration system, and Ammonium-Chloride-Potassium (ACK) buffer; (d) washing the PBMCs via centrifugation; (e) optionally cryopreserving the PBMCs; (f) optionally thawing the PBMCs if the PBMCs are cryopreserved in step (e); (g) washing the PBMCs using a membrane filtration system, e.g., a spinning membrane filtration system; (h) manufacturing the cells from the PBMCs; and (i) washing the cells using a membrane filtration system, e.g., a spinning membrane filtration system. In a specific embodiment, steps (a)-(d) and (g)-(i) are performed in order. In a specific embodiment, steps (a)-(i) in said method are performed in order.

In another embodiment, provided herein is a method of manufacturing chimeric antigen receptor (CAR)-expressing T cells (CAR T cells) from peripheral blood mononuclear cells (PBMCs) from a subject from which a blood sample is obtained, comprising the steps of: (a) obtaining PBMCs from the blood sample; (b) isolating PBMCs that have been obtained from the blood sample using a membrane filtration system and Ammonium-Chloride-Potassium (ACK) buffer; (c) washing the PBMCs via centrifugation; (d) optionally cryopreserving the PBMCs; (e) optionally thawing the PBMCs cryopreserved in step (d); (f) washing the PBMCs using a membrane filtration system; (g) manufacturing the CAR T cells from the PBMCs from step (f); and (h) washing the CAR T cells from step (g) using a membrane filtration system. In a specific embodiment, steps (a)-(h) in said method are performed in order.

In another embodiment, provided herein is a method of manufacturing chimeric antigen receptor (CAR)-expressing T cells (CAR T cells) from peripheral blood mononuclear cells (PBMCs) from a subject from which a blood sample is obtained, comprising the steps of: (a) isolating PBMCs that have been obtained from a blood sample form the subject, wherein the said isolating uses a membrane filtration system and Ammonium-Chloride-Potassium (ACK) buffer; (b) washing the PBMCs via centrifugation; (c) optionally cryopreserving the PBMCs; (d) optionally thawing the PBMCs cryopreserved in step (c); (e) washing the PBMCs using a membrane filtration system; (f) manufacturing the CAR T cells from the PBMCs from step (f); and (g) washing the CAR T cells from step (f) using a membrane filtration system. In a specific embodiment, steps (a)-(g) in said method are performed in order.

In another embodiment, provided herein is a method of manufacturing cells from peripheral blood mononuclear cells (PBMCs) from a blood sample from a subject, comprising the steps of: (a) isolating PBMCs from a blood sample from the subject, wherein the blood sample is a leukapheresed blood sample or is from a blood draw, using a membrane filtration system and Ammonium-Chloride-Potassium (ACK) buffer; (b) optionally cryopreserving the PBMCs from step (a); (c) optionally thawing the PBMCs from step (b); (d) washing the thawed PBMCs from step (c) using a membrane filtration system; (e) manufacturing the cells from the PBMCs from step (d) ; and (f) washing the cells from step (e) using a membrane filtration system. In a specific embodiment, steps (a)-(f) in said method are performed in order. In a specific embodiment, the cells are chimeric antigen receptor (CAR)-expressing T cells (CAR T cells).

In another embodiment, provided herein is a method of manufacturing cells from peripheral blood mononuclear cells (PBMCs) from a blood sample from a subject, comprising the steps of: (a) isolating PBMCs from a blood sample from the subject, wherein the blood sample is a leukapheresed blood sample or is from a blood draw, using a membrane filtration system and Ammonium-Chloride-Potassium (ACK) buffer; (b) cryopreserving the PBMCs from step (a); (c) thawing the PBMCs from step (b); (d) washing the thawed PBMCs from step (c) using a membrane filtration system; (e) manufacturing the cells from the PBMCs from step (d) ; and (f) washing the cells from step (e) using a membrane filtration system. In a specific embodiment, steps (a)-(f) in said method are performed in order. In a specific embodiment, the cells are chimeric antigen receptor (CAR)-expressing T cells (CAR T cells).

In another embodiment, provided herein is a method of manufacturing cells from peripheral blood mononuclear cells (PBMCs) from a blood sample from a subject, comprising the steps of: (a) isolating PBMCs from a blood sample from the subject using a membrane filtration system and Ammonium-Chloride-Potassium (ACK) buffer; (b) optionally cryopreserving and thawing the PBMCs from step (a); (c) optionally washing the thawed PBMCs from step (b) using a membrane filtration system; (d) manufacturing the cells from the PBMCs; and (e) washing the cells using a membrane filtration system. In a specific embodiment, the blood sample is a leukapheresed blood sample. In a specific embodiment, steps (a)-(e) in said method are performed in order.

In another embodiment, provided herein is a method of manufacturing cells from peripheral blood mononuclear cells (PBMCs) from a blood sample from a subject, comprising the steps of: (a) isolating PBMCs from a blood sample from the subject using a membrane filtration system and Ammonium-Chloride-Potassium (ACK) buffer; (b) cryopreserving and thawing the PBMCs from step (a); (c) washing the thawed PBMCs from step (b) using a membrane filtration system; (d) manufacturing the cells from the PBMCs; and (e) washing the cells using a membrane filtration system. In a specific embodiment, the blood sample is a leukapheresed blood sample. In a specific embodiment, steps (a)-(e) in said method are performed in order.

In a specific embodiment, provided herein is a method of preparing peripheral blood mononuclear cells (PBMCs) from a subject from which a blood sample is obtained for manufacturing chimeric antigen receptor (CAR)-expressing T cells (CAR T cells), comprising the steps of: (a) obtaining blood cells from the blood sample, e.g., using leukapheresis or blood draw to obtain peripheral blood mononuclear cells (PBMCs); (b) optionally freezing the blood cells and obtaining the PBMCs after thaw, or optionally obtaining and freezing the PBMCs, followed by thawing prior to subsequent steps; (c) isolating the PBMCs using a membrane filtration system, e.g., a spinning membrane filtration system, and Ammonium-Chloride-Potassium (ACK) buffer; (d) washing the PBMCs via centrifugation; (e) optionally cryopreserving the PBMCs; (f) optionally thawing the PBMCs if the PBMCs are cryopreserved in step (e); and (g) washing the PBMCs using a membrane filtration system, e.g., a spinning membrane filtration system. In a specific embodiment, steps (a)-(d) are performed in order. In a specific embodiment, steps (a)-(g) in said method are performed in order.

In another embodiment, provided herein is a method of preparing peripheral blood mononuclear cells (PBMCs) from a subject from which a blood sample is obtained for manufacturing chimeric antigen receptor (CAR)-expressing T cells (CAR T cells), comprising the steps of: (a) obtaining PBMCs from the blood sample; (b) isolating PBMCs that have been obtained from the blood sample using a membrane filtration system and Ammonium-Chloride-Potassium (ACK) buffer; (c) washing the PBMCs via centrifugation; (d) optionally cryopreserving the PBMCs; (e) optionally thawing the PBMCs cryopreserved in step (d); and (f) washing the PBMCs using a membrane filtration system. In a specific embodiment, steps (a)-(f) in said method are performed in order.

In another embodiment, provided herein is a method of preparing peripheral blood mononuclear cells (PBMCs) from a subject from which a blood sample is obtained for manufacturing chimeric antigen receptor (CAR)-expressing T cells (CAR T cells), comprising the steps of: (a) isolating PBMCs that have been obtained from a blood sample form the subject, wherein the said isolating uses a membrane filtration system and Ammonium-Chloride-Potassium (ACK) buffer; (b) washing the PBMCs via centrifugation; (c) optionally cryopreserving the PBMCs; (d) optionally thawing the PBMCs cryopreserved in step (c); and (e) washing the PBMCs using a membrane filtration system. In a specific embodiment, steps (a)-(e) in said method are performed in order.

In another embodiment, provided herein is a method of preparing peripheral blood mononuclear cells (PBMCs) from a subject from which a blood sample is obtained for manufacturing chimeric antigen receptor (CAR)-expressing T cells (CAR T cells), comprising the steps of: (a) isolating PBMCs from a blood sample from the subject, wherein the blood sample is a leukapheresed blood sample or is from a blood draw, using a membrane filtration system and Ammonium-Chloride-Potassium (ACK) buffer; (b) optionally cryopreserving the PBMCs from step (a); (c) optionally thawing the PBMCs from step (b); and (d) washing the thawed PBMCs from step (c) using a membrane filtration system. In a specific embodiment, steps (a)-(d) in said method are performed in order.

In another embodiment, provided herein is a method of preparing peripheral blood mononuclear cells (PBMCs) from a subject from which a blood sample is obtained for manufacturing chimeric antigen receptor (CAR)-expressing T cells (CAR T cells), comprising the steps of: (a) isolating PBMCs from a blood sample from the subject, wherein the blood sample is a leukapheresed blood sample or is from a blood draw, using a membrane filtration system and Ammonium-Chloride-Potassium (ACK) buffer; (b) cryopreserving the PBMCs from step (a); (c) thawing the PBMCs from step (b); and (d) washing the thawed PBMCs from step (c) using a membrane filtration system. In a specific embodiment, steps (a)-(d) in said method are performed in order.

In another embodiment, provided herein is a method of preparing peripheral blood mononuclear cells (PBMCs) from a subject from which a blood sample is obtained for manufacturing chimeric antigen receptor (CAR)-expressing T cells (CAR T cells), comprising the steps of: (a) isolating peripheral blood mononuclear cells (PBMCs) from a blood sample from the subject using a membrane filtration system and Ammonium-Chloride-Potassium (ACK) buffer; (b) optionally cryopreserving and thawing the PBMCs from step (a); and (c) optionally washing the thawed PBMCs from step (b) using a membrane filtration system. In a specific embodiment, the blood sample is a leukapheresed blood sample. In a specific embodiment, steps (a)-(c) in said method are performed in order.

In another embodiment, provided herein is a method of preparing peripheral blood mononuclear cells (PBMCs) from a subject from which a blood sample is obtained for manufacturing chimeric antigen receptor (CAR)-expressing T cells (CAR T cells), comprising the steps of: (a) isolating peripheral blood mononuclear cells (PBMCs) from a blood sample from the subject using a membrane filtration system and Ammonium-Chloride-Potassium (ACK) buffer; (b) cryopreserving and thawing the PBMCs from step (a); and (c) washing the thawed PBMCs from step (b) using a membrane filtration system. In a specific embodiment, the blood sample is a leukapheresed blood sample. In a specific embodiment, steps (a)-(c) in said method are performed in order.

For any of the above embodiments, T cells, NK cells or dendritic cells may be isolated from the PBMCs prior to formulation in solution. In more specific embodiments, the cells of any of the above methods are chimeric antigen receptor-expressing T cells (CAR T cells). For T cells, magnetic beads, such as Dynabeads, coated with anti-CD3, anti-CD4 or anti-CD8 antibodies may be used for T cell isolation.

Various aspects of the manufacturing process are discussed in more detail below.

### 4.2. Cryopreservation

PBMCs or cells, e.g., T cells, undergoing the cell manufacturing process, *e.g.,* the CAR T cell manufacturing process, described herein may be cryopreserved. As used herein, "cryopreserving" means the preservation of cells by cooling to sub-zero temperatures, *e.g*., to about or just under, the boiling point of liquid nitrogen, or -196°C. Cryoprotective agents are preferably used at sub-zero temperatures to prevent the cells being preserved from damage due to freezing at low temperatures or warming to room temperature. Cryoprotective agents which can be used include, but are not limited to, dimethyl sulfoxide (DMSO) (Lovelock and Bishop, Nature, 1959; 183: 1394-1395; Ashwood-Smith, Nature, 1961; 190: 1204-1205), glycerol, polyvinylpyrrolidine (Rinfret, Ann. N.Y. Acad. Sci., 1960; 85: 576), and polyethylene glycol (Sloviter and Ravdin, Nature, 1962; 196: 48). A preferred cooling rate is ~1°C to 3°C per minute. After at least two hours, the T cells have reached a temperature of -80°C and can be placed directly into liquid nitrogen (-196°C) for permanent storage such as in a long-term cryogenic storage vessel.

### 4.3. Culture Initiation And Stimulation

T cells, e.g., unmodified T cells, or T cells expressing CD3 and CD28, or comprising a polypeptide comprising a CD3ζ signaling domain and a CD28 co-stimulatory domain, can be expanded using antibodies to CD3 and CD28, e.g., antibodies attached to beads; see, e.g., U.S. Patent Nos. 5,948,893; 6,534,055; 6,352,694; 6,692,964; 6,887,466; and 6,905,681. Magnetic beads, such as Dynabeads, coated with anti-CD3 and anti-CD28 antibodies may be used for T cell expansion.

### 4.4. Chimeric Antigen Receptors

In certain embodiments, the PBMCs, e.g., immune cells, more specifically, T cells, produced during performance of the method described herein express one or more chimeric antigen receptors (CARs) on their surface. Generally, CARs comprise an extracellular domain from a first protein (e.g., an antigen-binding protein), a transmembrane domain, and an intracellular signaling domain, *e.g.,* a primary signaling domain and optionally one or more costimulatory domains. In preferred embodiments, once the extracellular domain binds to a target protein such as a tumor-associated antigen (TAA) or tumor-specific antigen (TSA), a signal is generated via the intracellular signaling domain that activates the immune cell, e.g., to target and kill a cell expressing the target protein.

Extracellular domains: The extracellular domains of the CARs bind to an antigen of interest. In certain embodiments, the extracellular domain of the CAR comprises a receptor, or a portion of a receptor, that binds to said antigen. In certain embodiments, the extracellular domain comprises, or is, an antibody or an antigen-binding portion thereof. In specific embodiments, the extracellular domain comprises, or is, a single chain Fv (scFv) domain. The single-chain Fv domain can comprise, for example, a V*_{L}* linked to V*_{H}* by a flexible linker, wherein said V*_{L}* and V*_{H}* are from an antibody that binds said antigen.

In certain embodiments, the antigen recognized by the extracellular domain of a polypeptide described herein is a tumor-associated antigen (TAA) or a tumor-specific antigen (TSA). In various specific embodiments, the tumor-associated antigen or tumor-specific antigen is, without limitation, Her2, prostate stem cell antigen (PSCA), alpha-fetoprotein (AFP), carcinoembryonic antigen (CEA), cancer antigen-125 (CA-125), CA19-9, calretinin, MUC-1, B cell maturation antigen (BCMA), epithelial membrane protein (EMA), epithelial tumor antigen (ETA), tyrosinase, melanoma-24 associated antigen (MAGE), CD19, CD22, CD27, CD30, CD34, CD45, CD70, CD99, CD117, EGFRvIII (epidermal growth factor variant III), mesothelin, PAP (prostatic acid phosphatase), prostein, TARP (T cell receptor gamma alternate reading frame protein), Trp-p8, STEAPI (six-transmembrane epithelial antigen of the prostate 1), chromogranin, cytokeratin, desmin, glial fibrillary acidic protein (GFAP), gross cystic disease fluid protein (GCDFP-15), HMB-45 antigen, protein melan-A (melanoma antigen recognized by T lymphocytes; MART-I), myo-D1, muscle-specific actin (MSA), neurofilament, neuron-specific enolase (NSE), placental alkaline phosphatase, synaptophysis, thyroglobulin, thyroid transcription factor-1, the dimeric form of the pyruvate kinase isoenzyme type M2 (tumor M2-PK), an abnormal ras protein, or an abnormal p53 protein. In certain other embodiments, the TAA or TSA recognized by the extracellular domain of a CAR is integrin αvβ3 (CD61), galactin, or Ral-B.

In certain embodiments, the TAA or TSA recognized by the extracellular domain of a CAR is a cancer/testis (CT) antigen, e.g., BAGE, CAGE, CTAGE, FATE, GAGE, HCA661, HOM-TES-85, MAGEA, MAGEB, MAGEC, NA88, NY-ES0-1, NY-SAR-35, OY-TES-1, SPANXBI, SPA17, SSX, SYCPI, or TPTE.

In certain other embodiments, the TAA or TSA recognized by the extracellular domain of a CAR is a carbohydrate or ganglioside, e.g., fuc-GMI, GM2 (oncofetal antigen-immunogenic-1; OFA-I-1); GD2 (OFA-I-2), GM3, GD3, and the like.

In certain other embodiments, the TAA or TSA recognized by the extracellular domain of a CAR is alpha-actinin-4, Bage-1, BCR-ABL, Bcr-Abl fusion protein, beta-catenin, CA 125, CA 15-3 (CA 27.29\BCAA), CA 195, CA 242, CA-50, CAM43, Casp-8, cdc27, cdk4, cdkn2a, CEA, coa-l, dek-can fusion protein, EBNA, EF2, Epstein Barr virus antigens, ETV6-AML1 fusion protein, HLA-A2, HLA-All hsp70-2, KIAA0205, Mart2, Mum-1, 2, and 3, neo-PAP, myosin class I, OS-9, pml-RARα fusion protein, PTPRK, K-ras, N-ras, triosephosphate isomerase, Gage 3,4,5,6,7, GnTV, Herv-K-mel, Lage-1, NA-88, NY-Eso-1/Lage-2, SP17, SSX-2, TRP2-Int2, gp100 (Pmel17), tyrosinase, TRP-1, TRP-2, MAGE-l, MAGE-3, RAGE, GAGE-l, GAGE-2, p15(58), RAGE, SCP-1, Hom/Mel-40, PRAME, p53, HRas, HER-2/neu, E2A-PRL, H4-RET, IGH-IGK, MYL-RAR, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, 13-Catenin, Mum-1, p16, TAGE, PSMA, CT7, telomerase, 43-9F, 5T4, 791Tgp72, 13HCG, BCA225, BTAA, CD68\KP1, C0-029, FGF-5, G250, Ga733 (EpCAM), HTgp-175, M344, MA-50, MG7-Ag, MOV18, NB\70K, NY-C0-1, RCAS1, SDCCAG16, TA-90, TAAL6, TAG72, TLP, or TPS.

In a specific embodiment, the tumor-associated antigen or tumor-specific antigen is an AML-related tumor antigen, as described in S. Anguille et al, Leukemia (2012), 26, 2186-2196.

Other tumor-associated and tumor-specific antigens are known to those in the art.

In a specific embodiment wherein the antigen is BCMA, the chimeric antigen receptor is BCMA02 (*see* Chekmasova et al., Blood 126:3094 (2015)). In a more specific embodiment, the CAR T cell expressing BCMA02 is bb2121 or bb21217.

Receptors, antibodies, and scFvs that bind to TSAs and TAAs, useful in constructing chimeric antigen receptors, are known in the art, as are nucleotide sequences that encode them.

In certain specific embodiments, the antigen recognized by the extracellular domain of a chimeric antigen receptor is an antigen not generally considered to be a TSA or a TAA, but which is nevertheless associated with tumor cells, or damage caused by a tumor. In certain embodiments, for example, the antigen is, e.g., a growth factor, cytokine or interleukin, e.g., a growth factor, cytokine, or interleukin associated with angiogenesis or vasculogenesis. Such growth factors, cytokines, or interleukins can include, e.g., vascular endothelial growth factor (VEGF), basic fibroblast growth factor (bFGF), platelet-derived growth factor (PDGF), hepatocyte growth factor (HGF), insulin-like growth factor (IGF), or interleukin-8 (IL-8). Tumors can also create a hypoxic environment local to the tumor. As such, in other specific embodiments, the antigen is a hypoxia-associated factor, e.g., HIF-1α, HIF-1β, HIF-2α, HIF-2β, HIF-3α, or HIF-3β. Tumors can also cause localized damage to normal tissue, causing the release of molecules known as damage associated molecular pattern molecules (DAMPs; also known as alarmins). In certain other specific embodiments, therefore, the antigen is a DAMP, e.g., a heat shock protein, chromatin-associated protein high mobility group box 1 (HMGB 1), S100A8 (MRP8, calgranulin A), S100A9 (MRP 14, calgranulin B), serum amyloid A (SAA), or can be a deoxyribonucleic acid, adenosine triphosphate, uric acid, or heparin sulfate.

Transmembrane domain: In certain embodiments, the extracellular domain of the CAR is joined to the transmembrane domain of the polypeptide by a linker, spacer or hinge polypeptide sequence, e.g., a sequence from CD28 or a sequence from CTLA4. The transmembrane domain can be obtained or derived from the transmembrane domain of any transmembrane protein, and can include all or a portion of such transmembrane domain. In specific embodiments, the transmembrane domain can be obtained or derived from, e.g., CD8, CD16, a cytokine receptor, and interleukin receptor, or a growth factor receptor, or the like.

Intracellular signaling domains: In certain embodiments, the intracellular domain of a CAR is or comprises an intracellular domain or motif of a protein that is expressed on or proximal to the surface of T cells and triggers activation and/or proliferation of said T cells. Such a domain or motif is able to transmit a primary antigen-binding signal that is necessary for the activation of a T lymphocyte in response to the antigen's binding to the CAR's extracellular portion. Typically, this domain or motif comprises, or is, an ITAM (immunoreceptor tyrosine-based activation motif). ITAM-containing polypeptides suitable for CARs include, for example, the zeta CD3 chain (CD3ζ) or ITAM-containing portions thereof. In a specific embodiment, the intracellular domain is or comprises a CD3ζ intracellular signaling domain; a CD3ζ intracellular signaling domain may be referred to as a primary signaling domain. In other specific embodiments, the intracellular domain (primary signaling domain) is from a lymphocyte receptor chain, a TCR/CD3 complex protein, an Fc receptor subunit or an IL-2 receptor subunit.

In certain embodiments, the CAR additionally comprises one or more co-stimulatory domains or motifs, e.g., as part of the intracellular domain of the polypeptide. The one or more co-stimulatory domains or motifs can be, or can comprise, one or more of a co-stimulatory CD27 polypeptide sequence or domain, a co-stimulatory CD28 polypeptide sequence or domain, a co-stimulatory OX40 (CD134) polypeptide sequence or domain, a co-stimulatory 4-1BB (CD137) polypeptide sequence or domain, or a co-stimulatory inducible T-cell costimulatory (ICOS) polypeptide sequence or domain, or other costimulatory domain or motif, or any combination thereof.

The CAR may also comprise a T cell survival motif. The T cell survival motif can be any polypeptide sequence or motif that facilitates the survival of the T lymphocyte after stimulation by an antigen. In certain embodiments, the T cell survival motif is, or is derived from, CD3, CD28, an intracellular signaling domain of IL-7 receptor (IL-7R), an intracellular signaling domain of IL-12 receptor, an intracellular signaling domain of IL-15 receptor, an intracellular signaling domain of IL-21 receptor, or an intracellular signaling domain of transforming growth factor β (TGFβ) receptor.

### 4.5. T Cells (T Lymphocytes)

T cells produced by the methods provided herein may be naive T lymphocytes or MHC-restricted T lymphocytes. In certain embodiments, the T lymphocytes are tumor infiltrating lymphocytes (TILs). In certain embodiments, the T cells are cytotoxic T cells (cytotoxic T lymphocytes, or CTLs), CD4+ T cells, CD8+ T cells, T effector (T_{EFF}) cells, or T central memory (T_{CM}) cells.

The T cells may be NKT cells (natural killer T cells), which refers to CD1d- restricted T cells, which express a T cell receptor (TCR). Unlike conventional T cells that detect peptide antigens presented by conventional major histocompatibility (MHC) molecules, NKT cells recognize lipid antigens presented by CD1d, a non-classical MHC molecule. Two types of NKT cells are currently recognized. Invariant or type I NKT cells express a very limited TCR repertoire - a canonical α-chain (Vα24-Jα18 in humans) associated with a limited spectrum of β chains (Vβ11 in humans). A second population of NKT cells, called nonclassical or noninvariant type II NKT cells, display a more heterogeneous TCR αβ usage. Adaptive or invariant (type I) NKT cells may be identified with the expression of at least one or more of the following markers: TCR Vα24-Jα18, Vb11, CD1d, CD3, CD4, CD8, αGalCer, CD161 and/or CD56.

The T cells produced by the methods described herein may be genetically modified T cells, e.g., may be modified to express a polypeptide such as a T cell receptor (TCR) or chimeric antigen receptor (CAR), e.g., one of the CARs described in Section 4.4, above. The modified immune cells, *e.g.,* T cells, are preferably autologous to an individual to whom the modified immune cells are to be administered. In certain other embodiments, the modified immune cells are allogeneic to an individual to whom the modified immune cells are to be administered. Where allogeneic T cells are used to prepare modified T cells, it is preferable to select T cells that will reduce the possibility of graft-versus-host disease (GVHD) in the individual. For example, in certain embodiments, virus-specific T cells are selected for preparation of modified T cells; such T cells will be expected to have a greatly reduced native capacity to bind to, and thus become activated by, any recipient antigens. In certain embodiments, recipient-mediated rejection of allogeneic T cells can be reduced by co-administration to the host of one or more immunosuppressive agents, e.g., cyclosporine, tacrolimus, sirolimus, cyclophosphamide, or the like.

The modified immune cells, e.g., modified T cells, can optionally comprise a "suicide gene" or "safety switch" that enables killing of substantially all of the modified immune cells when desired. For example, the modified T cells, in certain embodiments, can comprise an HSV thymidine kinase gene (HSV-TK), which causes death of the modified T cells upon contact with gancyclovir. In another embodiment, the modified T cells comprise an inducible caspase, e.g., an inducible caspase 9 (iCaspase9), e.g., a fusion protein between caspase 9 and human FK506 binding protein allowing for dimerization using a specific small molecule pharmaceutical. See Straathof et al., Blood 105(11):4247-4254 (2005).

### 4.6. Manufacturing of CAR T Cells

In certain embodiments, any of the CARs can be introduced into any of the cells described herein utilizing any of the methods described herein. In a specific embodiment, the method comprises: activating the cells, e.g. T cells, expanding the cells, transducing the cells using a vector, e.g., a lentiviral vector, encoding a CAR. In a specific embodiment, the cells, e.g., T cells, are activated using anti-CD3 and anti-CD28 antibodies. In specific embodiments, the anti-CD3 and anti-CD28 antibodies are immobilized by binding to Fc receptors on endogenous antigen presenting cells, such as monocytes and dendritic cells. In specific embodiments, the cells, e.g., T cells, are expanded using a static bag and/or expansion using a WAVE bioreactor (GE Healthcare Life Sciences).

### 4.7. Dendritic Cells

Dendritic cells (DCs) produced by the methods disclosed herein may be identified as follows. In their immature state, DCs may be characterized by low levels of MHC proteins and B7 co-stimulatory molecules, and the ability to conduct phagocytosis and pinocytosis, and by the absence of the surface molecules CD83 and CD25. In the mature state, they may be characterized by an altered pattern of cell surface proteins, wherein the surface expression of some or all of the following molecules is increased: CD25, CD40, CD70, CD80, CD83, CD86, and MHC proteins. "Mature" DCs are different from "immature" DCs, in that the former are immunostimulatorily more active, usually retain the ability to migrate into the draining lymph nodes in vivo, and to present increasingly endogenously expressed and exogenous antigen in the MHC context. Under physiological conditions, only "mature" DCs are able to activate naive T cells.

### 4.8. Natural Killer Cells

Natural Killer (NK) cells produced by the methods provided herein may be defined by the expression of CD56 or CD16 and the absence of the T cell receptor (CD3). The NK cells may be "adaptive NK cells" or "memory NK cells," which terms are interchangeable, and which refer to a subset of NK cells that are phenotypically CD3- and CD56+, express NKG2C and CD57, and optionally, CD16, but lack expression of one or more of the following: PLZF, SYK, FceRy, and EAT-2. Isolated subpopulations of CD56+ NK cells produced by the methods provided herein may comprise expression of CD16, NKG2C, CD57, NKG2D, NCR ligands, NKp30, NKp40, NKp46, activating and/or inhibitory KIRs, NKG2A and DNAM-1. CD56+ may be dim or bright expression. The NK cells produced by the methods described herein may be genetically modified NK cells, e.g., may be modified to express a polypeptide such as a T cell receptor (TCR) or chimeric antigen receptor (CAR).

### 4.9. Expression Vectors and Cell Transduction

Typically, polynucleotide sequences that express a chimeric antigen receptor are carried into the cell (e.g., T cells or NK cells) in a polynucleotide vector. Vectors derived from retroviruses such as lentiviruses are suitable tools to achieve long-term gene transfer since they allow long-term, stable integration of a transgene and its propagation in daughter cells. Expression of natural or synthetic nucleic acids encoding CARs may be achieved by operably linking a nucleic acid encoding the CAR polypeptide or portions thereof to a promoter, and incorporating the construct into an expression vector suitable for expression in a eukaryotic cell, *e.g.,* a T cell.

The expression vector may generally be provided to a cell in the form of a viral vector. Viral vector technology is well known in the art and is described, for example, in Sambrook *et al.* (2001, Molecular Cloning: A Laboratory Manual, Cold Spring Harbor Laboratory, New York), and in other virology and molecular biology manuals. Viruses, which are useful as vectors include, but are not limited to, retroviruses, adenoviruses, adeno-associated viruses, herpes viruses, lentiviruses, poxviruses, herpes simplex virus I, and the like. See, for example, U.S. Pat. Nos. 5,350,674 and 5,585,362.

In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

A number of viral based systems have been developed for gene transfer into mammalian cells. For example, a selected gene can be inserted into a vector and packaged in retroviral particles using techniques known in the art. The recombinant virus can then be isolated and delivered to cells of the subject ex vivo. Alternatively, adenovirus vectors may be used.

A vector may comprise a promoter and optionally one or more promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, for example, the spacing between promoter elements can be increased to 50 bp apart before activity begins to decline. The immediate early cytomegalovirus (CMV) promoter sequence may be used to drive expression of the CAR. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the invention should not be limited to the use of constitutive promoters. Inducible promoters may also be used, including, but not limited to, a metallothionine promoter, a glucocorticoid promoter, a progesterone promoter, or a tetracycline promoter.

In order to assess the expression of a CAR polypeptide on the CAR T cell, the expression vector to be introduced into a cell can also contain either a selectable marker gene or a reporter gene or both to facilitate identification and selection of expressing cells from the population of cells sought to be transfected or infected through viral vectors. The selectable marker may be carried on a separate polynucleotide and co-transfected into the T cell along with the vector encoding the CAR. Selectable markers and reporter genes may be flanked with appropriate regulatory sequences to enable expression in the host cells. Useful selectable markers include, for example, antibiotic-resistance genes, such as neo and the like.

Reporter genes may be used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the polynucleotide encoding it has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into eukaryotic cells, *e.g.,* T cells, are known in the art. In the context of an expression vector, the vector can be readily introduced into a T cell by any method known in the art. Physical methods for introducing a polynucleotide, *e.g.,* vector encoding a CAR, into a host cell include calcium phosphate precipitation, lipofection, particle bombardment, microinjection, electroporation, or the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

### 5. EXAMPLES

### 5.1. Example 1: Improved Process for Manufacturing CAR-T Cells

### 5.1.1. Baseline Process

A baseline CAR T cell manufacturing process was developed for a BCMA-directed chimeric antigen receptor (CAR)-expressing T cell (*see* Hollyman et al., J. Immunother. 2009, 32:169-180). Manufacturing process parameters such as PBMC isolation, T cell activation, transduction, and expansion were initially developed and optimized using a small-scale, T flask based process. Once the process parameters were established and optimized, the process was scaled up for clinical manufacturing. The process began with leukapheresis, followed by isolation of peripheral blood mononuclear cells (PBMCs) by density gradient centrifugation using a Cell-Saver 5+ (CS5+; Haemonetics, Braintree, Massachusetts); a pre-culture wash using batch centrifugation culture of the resulting PBMC in TCGM-HABS medium (T Cell Growth Medium comprising X-VIVO-15^{™} chemically-defined hematopoietic cell medium (Lonza, Basel, Switzerland) without phenol red, supplemented with 5% v/v human AB serum 2mM GlutaMAX^{™} (Gibco), 10mM HEPES (4-(2-hydroxyethyl)-1-piperazineethansulfonic acid (Thermo Fisher Scientific), and 300 IU/mL recombinant human interleukin-2 (rhIL2)). The same medium, supplemented further with 1% sodium pyruvate and 1% minimal essential vitamins was also tested and found to result in an equivalent number of population doublings over eight days. For simplicity, TCGM-HABS was selected for the process. After comparison with additional media, the concentration of rhIL2 was reduced from 300 IU/mL to 100IU/mL.

T cells within the PBMC were then activated using soluble anti-CD3 and anti-CD28 antibodies immobilized by binding to Fc receptors on endogenous antigen presenting cells, such as monocytes and dendritic cells. Activation of T cells was confirmed by determining the size of the cells; resting T cells have a volume of ~180-200 fL, while strongly activated T cells increase in size to >500 fL. The activation step was followed by expansion in a static bag followed by expansion in a WAVE bioreactor (GE Healthcare Life Sciences). Expansion was followed by transduction of the expanded T cells with a lentiviral vector encoding the BCMA-targeting CAR Based on studies utilizing a range of multiplicities of infection from 0.625 to 40, and monitoring population doublings, cell size, and expression of the anti-BCMA CAR, a range of 10-30 MOI was used for the base clinical manufacturing process. The resulting cells were harvested and washed post-harvest wash using a CD5+ device.

The initial steps in the baseline process, from leukapheresis to T cell activation, however, required some labor-intensive steps, in particular, the initial PBMC isolation and the density gradient centrifugation-dependent wash were amended to improve efficiency.

### 5.1.2. Improved Process

Consistency of the T cell manufacturing process is key to consistency in producing CAR T cell drug product. To this end, the baseline process was sought to be improved by substituting a LOVO-ACK isolation step, LOVO pre-culture initiation wash, and final LOVO wash step, in place of the baseline process's CS5+ device-dependent steps.

The LOVO-ACK PBMC isolation process consisted of two steps performed in sequence: a wash using the LOVO Automated Cell Processing System (Fresenius Kabi, Lake Zurich, Illinois; referred to herein as "LOVO"), followed by ACK (ammonium-chloride-potassium) lysing buffer incubation. ACK buffer is 150 mM NH₄Cl, 10 mM KHCO₃, and 0.1 mM sodium EDTA in water (e.g., deionized water). The LOVO wash step uses the LOVO Automated Cell Processing System, which features a spinning membrane filtration system designed to effectively remove platelets and cell debris while retaining the PBMC populations in the starting leukapheresis material. The wash step takes place within a closed pre-sterilized single-use LOVO disposable kit which features a cell separation device (spinning membrane filtration system) to facilitate the removal of platelets, cell debris, and plasma.

After the LOVO wash step, ACK buffer was added to the remaining cell suspension in order to lyse red blood cells. This step was performed in a transfer bag with a cell contact surface composed of polyvinyl chloride. The RBC-depleted PBMC were then separated from the ACK buffer solution and washed via centrifugation.

The isolated and washed PBMC were then optionally cryopreserved (if to be shipped prior to use) using standard cryopreservation techniques and controlled-rate freezing. The cells were thawed in a 37° bath immediately prior to subsequent use. Over three test lots, cryopreserved cells produced adequate numbers of T cells, and after CAR T manufacturing, CAR + T cells.

| **PBMC lot** | **Total T Cells Manufactured** | **% CAR+** | **Total CAR+ T Cells** |
|---|---|---|---|
| 3101 | 1.32 × 10¹⁰ | 29.4% | 3.88 × 10⁹ |
| 3167 | 6.05 × 10⁹ | 22.5% | 1.36 × 10⁹ |
| 3219 | 5.76 × 10⁹ | 23.8% | 1.37 × 10⁹ |

After thawing, the PBMCs underwent a cell culture process comprising culture initiation and stimulation with anti-CD3 and anti-CD28 antibodies (Day 0); T cell transduction with a lentiviral vector carrying coding sequences for the anti-BCMA chimeric antigen receptor (Day 1); cell counting and reseeding in gas permeable cell culture bags (Days 2-5); cell counting and reseeding in a WAVE^{™} Bioreactor (Days 6-9); followed by cell harvest and a final wash using the LOVO device (Day 10).

The final steps in the improved LOVO-ACK process are therefore as follows:
a. Leukapheresis;
b. LOVO-ACK PBMC isolation;
c. Washing of PBMCs via centrifugation;
d. PBMC cryopreservation (if to be shipped);
e. PBMC thaw (if shipped);
f. PBMC wash using LOVO
g. T cell activation, expansion, and cell harvest;
h. Wash using LOVO.

The improved process had the advantages over the baseline process of closed process steps (reducing the probability of contamination); enhanced PBMC preparation robustness and reproducibility; reduced drug product processing time; and reduction in overall process complexity.

### 5.2. Example 2: Improved Process Superiority Studies

### Comparison of Baseline Isolation Process and LOVO-ACK Isolation Process

This Example shows that, for some parameters, the improved process is superior to the baseline process.

A feasibility study comparing the baseline CS5+ PBMC isolation process with the LOVO-ACK PBMC isolation process was conducted with leukapheresis units from 3 healthy donors and 2 multiple myeloma (MM) subjects. Following side-by-side PBMC isolation with the baseline process and LOVO ACK process, isolated PBMCs were cryopreserved and processed using the same cell culture, DP formulation, and cryopreservation methods. As summarized below, PBMC viability and phenotypic composition, PBMC CD4+ and CD8+ T cell subsets, cell culture characterization, and drug product release test results demonstrated that the LOVO-ACK isolation process is a feasible replacement for the CS5+ PBMC isolation procedure.

### PBMC Isolation & Recovery

The PBMC isolation step is designed to remove red blood cells (RBCs) and platelets (PLTs) from the leukapheresis starting material. As illustrated in Table 1, the updated PBMC isolation method resulted in more extensive and consistent reduction of red blood cells (RBCs) and depletion of platelets (PLTs), therefore improving the quality of PBMCs for cell culture initiation.

**Table 1 - Comparison of Pre- to Post- Change PBMC RBC/WBC & PLT/WBC**

| **Donor Lot** | **PBMC RBCs Removal** | | | **PBMC PLTs Removal** | | |
|---|---|---|---|---|---|---|
| | Baseline Process (RBC/W BC) | Updated Process (RBC/W BC) | Pre- to Post-Change Difference | Baseline Process (PLT/WB C) | Updated Process (PLT/WB C) | Pre- to Post-Change Difference |
| 1 | 4 | 1 | -3 | 18 | 1 | -17 |
| 2 | 5 | 2 | -3 | 28 | 1 | -27 |
| 3 | 4 | 2 | -2 | 19 | 1 | -18 |
| 4 | 4 | 2 | -2 | 36 | 0 | -36 |
| 5 | 2 | 1 | -1 | 13 | 0 | -13 |
| Mean | 4 | 2 | -2 | 23 | 1 | -22 |

| | | | | | | |
|---|---|---|---|---|---|---|
| Values = percentages of total cells | | | | | | |

As the two PBMC processes utilized different devices for PBMC isolation, the cell yield obtained from the PBMC isolation step has been assessed and compared. As shown in Table 2, the PBMC recoveries of the baseline and updated processes yields were highly similar, with a mean pre- to post- change difference of -2 percentage points.

**Table 2 - Comparison of PBMC Isolation Recoveries**

| **Donor Lot** | **PBMC Isolation Recovery** | | |
|---|---|---|---|
| | Baseline Process (%) | Updated Process (%) | Pre- to Post- Change Difference |
| 1 | 82 | 86 | 4 |
| 2 | 70 | 73 | 2 |
| 3 | 87 | 78 | -9 |
| 4 | 61 | 59 | -2 |
| 5 | 73 | 69 | -4 |
| Mean | 75 | 73 | -2 |

Thus, the improved process showed PBMC isolation and recovery, and RBC removal; and superior platelet removal, as compared to the baseline process.

In-Process Cell Culture Characterization - T Cell Growth & Peak T Cell Activation Comparison

The cell culture characterization results are presented in Table 3. T cells manufactured from the baseline and updated processes displayed comparable growth kinetics, as evidenced by the similar extent of T cell growth (mean pre- to post- change difference in T cell growth is -1 population doubling) and activation profile (mean pre- and post- change peak activation day in cell culture being the same).

**Table 3 - T Cell Growth & Peak T Cell Activation Day in Cell Culture**

| **BCMA CAR T cell Lot** | **T Cell Growth (T cell PDL at Harvest)** | | | **Peak Activation Time (Day in Cell Culture)** | | |
|---|---|---|---|---|---|---|
| | Baseline Process | Updated Process | Pre- to Post-Change Difference | Baseline Process | Updated Process | Pre- to Post-Change Difference |
| 1 | 8 | 7 | -1 | Day 3 | Day 3 | 0 day |
| 2 | 11 | 11 | 0 | Day 4 | Day 4 | 0 day |
| 3 | 7 | 7 | 0 | Day 5 | Day 4 | -1 day |
| 4 | 9 | 8 | -1 | Day 4 | Day 4 | 0 day |

**Table 3 - T Cell Growth & Peak T Cell Activation Day in Cell Culture**

| **BCMA CAR T cell Lot** | **T Cell Growth (T cell PDL at Harvest)** | | | **Peak Activation Time (Day in Cell Culture)** | | |
|---|---|---|---|---|---|---|
| | Baseline Process | Updated Process | Pre- to Post-Change Difference | Baseline Process | Updated Process | Pre- to Post-Change Difference |
| 5 | 9 | 8 | -1 | Day 3 | Day 4 | 1 day |
| Mean | 9 | 8 | -1 | Day 4 | Day 4 | 0 day |

In-Process Drug Product Characterization Results - Post-Harvest Wash Recoveries

As the two drug product processes utilized different devices for the washing step after Day 10 harvest, the cell yield obtained from the post-harvest wash step has been assessed and compared. As shown in Table 4, the post-harvest wash recoveries of drug product (CAR T cells) resulting from the updated process were noticeably improved over the baseline process, with an observed mean increase of 30 percent points.

**Table 4 - Post-Harvest Wash Recoveries**

| **PBMC Lot ID** | **Post-Harvest Wash Recovery** | | |
|---|---|---|---|
| | Baseline Process (%) | Updated Process (%) | Pre- to Post- Change Difference |
| 1 | 63 | 93 | 30 |
| 2 | 77 | 94 | 17 |
| 3 | 63 | 101^{∗} | 38 |
| 4 | 71 | 97 | 26 |
| 5 | 52 | 88 | 36 |
| Mean | 65 | 95 | 30 |

| | | | |
|---|---|---|---|
| ^{∗}Value in excess of 100 due to measurement error. | | | |

Thus, the improved process shows markedly superior recovery of CAR T cells, as compared to the baseline process.

### 5.3. Example 3: Improved Process Comparability Studies

This Example demonstrates that, with respect to other parameters, the improved process is comparable to the baseline process.

The baseline and improved PBMC isolation processes were compared and determined to produce equivalent results, as follows. The process change conditions are outlined in Table 5. For each healthy donor, the leukapheresis unit was divided evenly for the baseline CS5+ device and updated "LOVO-ACK" PBMC isolation methods. Following PBMC isolation step, the PBMC lots were either cryopreserved or were used to initiate cell culture without cryopreservation. Prior to cell culture initiation, PBMCs were washed by batch centrifugation or the LOVO cell processing device. Gas permeable cell culture bags were used throughout the duration of cell culture. At day 10, post-harvest drug substance was washed with either the CS5+ device or the LOVO cell processing device. Formulated drug product was cryopreserved in bags for non-clinical in vivo comparability assessment and post-thaw in-use stability assay, or in vials for *in vitro* comparability assessment.

**Table 5 - Process change conditions for comparability assessment**

| **Condition ID** | **Description** | **Process Conditions** | | |
|---|---|---|---|---|
| | | **Process Step: PBMC Preparation** | | **Process Step: Drug Product Formulation & Cryopreservation** |
| | | **Operation: PBMC Isolation** | **Operation: Pre-Cell Culture Initiation Wash** | **Operation: Post-Harvest Wash** |
| **1** | **Baseline Process** | Ficoll density centrifugation w/ CS5+ Device | Batch centrifugation | CS5+ Device |
| **2** | **Updated Process** | LOVO-ACK method | • N/A (part of LOVO-ACK method) if no PBMC holding step | LOVO cell processing device |
| | | | • LOVO cell processing device if PBMC freeze holding step | |

The comparability study was conducted with five healthy donor leukapheresis units, from which ten anti-BCMA CAR T cell lots were generated (pre- and post-method change for each).

Each of the starting leukapheresis units were divided and processed for PBMC isolation with the baseline and updated processes in parallel. Three of the five PBMC lots were cryopreserved, followed by thaw and cell culture initiation, while the remaining two PBMC lots were immediately processed for cell culture initiation without freezing. The comparability of cryopreserved (PBMC holding step) and fresh (no PBMC holding step) PBMC in the anti-BCMA CAR T cell manufacturing process was previously established. At the end of cell culture, the baseline and updated processes continued with harvest and drug product processing steps, incorporating their respective cell washing methods. In-process and cryopreserved drug product test samples generated with the baseline and updated processes were assessed according to the comparative analytical testing plan as described below.

### Comparative Analytical Tests

Results of the PBMC viability comparison are provided in Table 6. There was no consistent difference in viability, with the mean pre- to post- change difference being +2 percent point.

**Table 6 - Comparison of Pre- to Post- Change PBMC Viability**

| **PBMC Lot** | **PBMC Viability** | | |
|---|---|---|---|
| | Baseline Process (%) | Improved Process (%) | Pre- to Post- Change Difference |
| 1 | 95 | 97 | 2 |
| 2 | 95 | 97 | 2 |
| 3 | 97 | 97 | 0 |
| 4 | 95 | 96 | 1 |
| 5 | 95 | 96 | 1 |
| Mean | 95 | 97 | 2 |

### PBMC Composition Comparison

Results of PBMC composition comparison were provided in Table 7 for CD45+ leukocytes, CD3+CD56- T cells, CD14+ monocytes and Table 8 for CD19+ B cells, CD3-CD56+ NK cells, and CD56-CD16+ granulocytes, respectively. The PBMC compositions were comparable, as the mean pre- to post- change differences in CD45+ leukocytes, CD3+CD56- T cells, CD14+ monocytes, CD19+ B cells, and CD3-CD56+ NK cells were +1, +2, +1, -1, and -1 percent points respectively. There was no significant presence of CD56-CD16+ granulocytes (not detected) with either processes. Taken together, the PBMC compositions were consistent across the baseline and the updated processes.

**Table 7 - PBMC Composition: CD45+ Leukocytes, CD3+CD56- T Cells, CD14+ Monocytes**

| **PBMC Lot** | **CD45+ leukocytes** | | | **CD3+CD56- T Cells** | | | **CD14+ monocytes** | | |
|---|---|---|---|---|---|---|---|---|---|
| | Gated on viable cells | | | Gated on CD45+ leukocyte | | | Gated on CD45+ leukocytes | | |
| | Baselin e Process (%) | Updat ed Proces s (%) | Pre- to Post-Change Differe nce | Baseli ne Proces s (%) | Updat ed Proces s (%) | Pre- to Post-Change Differe nce | Baseli ne Proces s (%) | Updat ed Proces s (%) | Pre- to Post-Change Differe nce |
| 1 | 95 | 97 | 2 | 57 | 61 | 4 | 20 | 17 | -3 |
| 2 | 98 | 97 | -1 | 41 | 48 | 7 | 28 | 24 | -4 |
| 3 | 96 | 96 | 0 | 41 | 36 | -5 | 31 | 40 | 9 |
| 4 | 94 | 96 | 2 | 20 | 20 | 0 | 42 | 45 | 3 |
| 5 | 95 | 97 | 2 | 43 | 45 | 2 | 30 | 31 | 1 |
| Mean | 96 | 97 | 1 | 40 | 42 | 2 | 30 | 31 | 1 |

**Table 8 - PBMC Composition: CD19+ B cells, CD3-CD56+ NK Cells, CD56-CD16+ Granulocytes**

| **PBMC Lot** | **CD19+ B cells** | | | **CD3-CD56+ NK Cells** | | | **CD56-CD16+ granulocytes** | | |
|---|---|---|---|---|---|---|---|---|---|
| | Gated on viable cells | | | Gated on CD45+ leukocytes | | | Gated on CD45+ leukocytes | | |
| | Baselin e Proces s (%) | Update d Proces s (%) | Pre- to Post-Change Differe nce | Baseli ne Proces s (%) | Updat ed Proces s (%) | Pre- to Post-Change Differe nce | Baseli ne Proces s (%) | Updat ed Proces s (%) | Pre- to Post-Chang e Differe nce |
| 1 | 7 | 9 | 2 | 10 | 9 | -1 | 0 | 0 | 0 |
| 2 | 16 | 15 | -1 | 8 | 7 | -1 | 0 | 0 | 0 |
| 3 | 11 | 6 | -5 | 9 | 10 | 1 | 0 | 0 | 0 |
| 4 | 8 | 7 | -1 | 21 | 21 | 0 | 0 | 0 | 0 |
| 5 | 7 | 6 | -1 | 11 | 11 | 0 | 1 | 0 | -1 |
| Mean | 10 | 9 | -1 | 12 | 11 | -1 | 0 | 0 | 0 |

### PBMC CD4+ & CD8+ T Cell Expression Comparison

The PBMC T cell population was further characterized for CD4+ and CD8+ expression. As shown in Table 9, the CD4+/CD8+ subset distribution of the PBMC T cells was comparable between the two processes, with the mean pre- to post- change difference for CD4+ and CD8+ cells within the T cell population being -3 and +3 percentage points, respectively.

**Table 9 - PBMC CD4+ & CD8+ T Cell Subsets**

| **PBMC Lot** | **CD4+ T cells** | | | **CD8+ T cells** | | |
|---|---|---|---|---|---|---|
| | Gated on CD3+CD56- T cells | | | Gated on CD3+CD56- T cells | | |
| | Baseline Process (%) | Updated Process (%) | Pre- to Post-Change Difference | Baseline Process (%) | Updated Process (%) | Pre- to Post-Change Difference |
| 1 | 63 | 60 | -3 | 29 | 34 | 5 |
| 2 | 69 | 68 | -1 | 27 | 28 | 1 |
| 3 | 68 | 66 | -2 | 24 | 24 | 0 |
| 4 | 68 | 65 | -3 | 26 | 28 | 2 |
| 5 | 50 | 45 | -5 | 41 | 47 | 6 |
| Mean | 64 | 61 | -3 | 29 | 32 | 3 |

Differences were observed between the normal donor PBMC leukocyte composition data generated for this process change comparability assessment, and the multiple myeloma (MM) patient PBMC data from a previous study. Compared to the healthy donors, the PBMCs of MM patients comprise lower numbers of B cells and T cells, and increased numbers of monocytes.

In addition, the CD4+/CD8+ ratio within the T cell population was different as well. Using the baseline process, the mean (N=5) percentages of B cells, T cells, and monocytes in healthy donor PBMC lots were 9.7% ± 4.2%, 40.6% ± 13.1%, 30.0% ± 7.8%, respectively. The mean (N=5) CD4+/CD8+ T cell ratio of the healthy donor PBMC lots was 58.8%/32.7% of T cells. Using the same baseline PBMC process, the mean (N=24) percentages of B cells, T cells, and monocytes in CRB-401 PBMC lots were 1.9% ± 1.5%, 29.9% ± 15.7%, 56.4% ± 19.4% respectively. The mean (N=24) CD4+/CD8+ T cell ratio of the CRB-401 PBMC lots was 40.8%/54.4% of T cells. Such observed differences in the two datasets were unrelated to the process change, and were consistent to those reported in scientific literature.

Thus, by the above parameters, the improved process is comparable to the baseline process.

### Embodiments:

1. A method of manufacturing chimeric antigen receptor (CAR)-expressing T cells (CAR T cells) from peripheral blood mononuclear cells (PBMCs) from a subject from which a blood sample is obtained, comprising the steps of:
   a. obtaining PBMCs from the blood sample;
   b. isolating the PBMCs that have been obtained from the blood sample using a membrane filtration system and Ammonium-Chloride-Potassium (ACK) buffer;
   c. washing the PBMCs via centrifugation;
   d. optionally cryopreserving the PBMCs;
   e. optionally thawing the PBMCs cryopreserved in step (d);
   f. washing the PBMCs using a membrane filtration system;
   g. manufacturing the CAR T cells from the PBMCs from step (f); and
   h. washing the CAR T cells from step (g) using a membrane filtration system.
2. A method of manufacturing chimeric antigen receptor (CAR)-expressing T cells (CAR T cells) from peripheral blood mononuclear cells (PBMCs) from a leukapheresed blood sample from a subject, comprising the steps of:
   a. isolating the PBMCs from the leukapheresed blood sample using a membrane filtration system and Ammonium-Chloride-Potassium (ACK) buffer;
   b. cryopreserving the PBMCs from step (a);
   c. thawing the PBMCs from step (b);
   d. washing the thawed PBMCs from step (c) using a spinning filtration system;
   e. manufacturing the CAR T cells from the PBMCs from step (d); and
   f. washing the CAR T cells from step (e) using a membrane filtration system.
3. A method of manufacturing chimeric antigen receptor (CAR)-expressing T cells (CAR T cells) from peripheral blood mononuclear cells (PBMCs) from a blood sample from a subject, comprising the steps of:
   a. isolating the PBMCs from the blood sample using a membrane filtration system and Ammonium-Chloride-Potassium (ACK) buffer;
   b. cryopreserving and thawing the PBMCs from step (a);
   c. washing the thawed PBMCs from step (b) using a membrane filtration system;
   d. manufacturing the CAR T cells from the PBMCs; and
   e. washing of the CAR T cells using a spinning membrane filtration system.
4. The method of any of embodiments 1-3, wherein any or all of the membrane filtration systems are a spinning membrane filtration system.
5. The method of embodiment 4, wherein the spinning membrane filtration system is a LOVO Automated Cell Processing System.
6. The method of any of embodiments 1-5, wherein the ACK buffer comprises 50-300 mM ammonium chloride, 5-25 mM potassium carbonate, and 0.05-0.25 mM sodium EDTA.
7. The method of embodiment 6, wherein said ACK buffer comprises 150 mM ammonium chloride, 10 mM potassium carbonate, and 0.1 mM sodium EDTA.
8. The method of any of embodiments 1-7, wherein said method improves reduction of platelets and red blood cells from said PBMCs by 18%-36% as compared to the same method using density gradient centrifugation in place of each use of membrane filtration.
9. The method of any of embodiments 1-8, wherein said method improves recovery of CAR T cells after CAR T cell manufacturing by 17%-36% as compared to the same method using density gradient centrifugation in place of each use of membrane filtration.

## Claims

1. A method of manufacturing chimeric antigen receptor (CAR)-expressing T cells from peripheral blood mononuclear cells (PBMCs) from a blood sample from a subject, comprising the steps of:
(a) isolating PBMCs from a blood sample from the subject using a membrane filtration system and Ammonium-Chloride-Potassium (ACK) buffer;
(b) manufacturing the CAR T cells from the PBMCs; and
(c) washing the cells using a spinning membrane filtration system.

2. The method of claim 1, wherein the PBMCs are separated from the ACK buffer solution and washed via centrifugation after step (a).

3. The method of claim 1 or 2, wherein T cells are isolated from the isolated PBMCs.

4. The method of claim 3, wherein magnetic beads coated with anti-CD3, anti-CD4 or anti-CD8 antibodies are used for the T cell isolation.

5. The method of any one of claims 1-4, wherein the T cells are cultured following their isolation.

6. The method of any one of claims 1-5, wherein the isolated PBMCs or T cells are cryopreserved.

7. The method of claim 6, wherein the T cells are thawed, washed, and resuspended in growth medium suitable for the growth of such cells.

8. The method of claim 7, wherein the thawed T cells are washed using the spinning membrane system or centrifugation prior to the cell culture of the thawed T cells.

9. The method of claim 1, 5 or 8, wherein manufacturing the CAR T cells comprises activating the T cells, expanding the T cells and transducing the T cells with a vector encoding a CAR.

10. The method of claim 9, wherein the vector is a lentiviral vector.

11. The method of claim 9, wherein the activating comprises using anti-CD3 and anti-CD28 antibodies.

12. The method of claim 9, wherein the CAR is an anti-BCMA CAR.

13. The method of any one of claims 1-14, wherein the spinning membrane filtration system has a pore size of 3.6 µm.

14. The method of claim 7, wherein the growth medium suitable for the growth of the T cells comprises 5% (v/v) human AB serum and 100 or 300 IU/mL IL-2.
